(19) **Europäisches Patentamt / European Patent Office / Office européen des brevets**

(11) **EP 3 386 596 B1**

(12) # EUROPEAN PATENT SPECIFICATION

(45) Date of publication and mention
of the grant of the patent:
**12.05.2021 Bulletin 2021/19**

(51) Int Cl.:
*A61Q 19/00* (2006.01)    *A61Q 19/08* (2006.01)
*A61Q 17/04* (2006.01)

(21) Application number: **16810484.2**

(86) International application number:
**PCT/IB2016/057427**

(22) Date of filing: **08.12.2016**

(87) International publication number:
**WO 2017/098426 (15.06.2017 Gazette 2017/24)**

(54) **TOPICAL SKIN CARE COMPOSITIONS COMPRISING MYRSINE AFRICANA EXTRACTS**

TOPISCHE HAUTPFLEGEZUSAMMENSETZUNGEN MIT MYRSINE-AFRICANA-EXTRAKTEN

COMPOSITIONS TOPIQUES DE SOIN DE LA PEAU CONTENANT DES EXTRAITS DE MYRSINE AFRICANA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.12.2015 ZA 201508992**

(43) Date of publication of application:
**17.10.2018 Bulletin 2018/42**

(73) Proprietor: **University Of Pretoria
0002 Pretoria (ZA)**

(72) Inventor: **LALL, Namrita
Silverlakes Golf Estate (ZA)**

(74) Representative: **D Young & Co LLP
120 Holborn
London EC1N 2DY (GB)**

(56) References cited:
**WO-A1-2014/020575      KR-A- 20100 132 245
US-A1- 2003 115 687**

- SADIQ AZAM ET AL: "Anti-spasmodic action of crude methanolic extract and a new compound isolated from the aerial parts of Myrsine africana", BMC COMPLEMENTARY AND ALTERNATIVE MEDICINE, BIOMED CENTRAL LTD., LONDON, GB, vol. 11, no. 1, 6 July 2011 (2011-07-06), page 55, XP021103512, ISSN: 1472-6882, DOI: 10.1186/1472-6882-11-55
- AHMAD BASHIR ET AL: "Anti-inflammatory activity and a new compound isolated from aerial parts of Myrsine africana", AFRICAN JOURNAL OF BIOTECHNOLOGY, vol. 10, no. 42, 8 August 2011 (2011-08-08), pages 8465-8470, XP055340782, DOI: 10.5897/AJB11.115
- Yan-Ping Zou ET AL: "Chemical Constituents from Myrsine africana L", , 1 January 2008 (2008-01-01), XP055340791, Retrieved from the Internet: URL:http://onlinelibrary.wiley.com/doi/10.1002/hlca.200890234/abstract [retrieved on 2017-01-31]

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

## BACKGROUND OF THE INVENTION

The present invention relates to topical formulations containing an ethanolic leaf extract of *Myrsine africana* and uses thereof. The extract produced according to the method disclosed herein exhibits inhibitory activity against the elastase enzyme in *in vitro* and *in vivo* studies.

*Myrsine africana* belonging to the Myrsinaceae family is a small shrub also known as the Cape Myrtle, African boxwood or Thakisa. The plant is indigenous to Macaronesia, Africa and South Asia. It is found throughout South Africa, commonly in areas where summer and winter rainfall occurs. It grows naturally on rocky krantzes and forests. *Myrsine africana* has dense, dark-green to red leaves and produces tiny bright purple berries. It can reach heights of over 2 meters and may be dense if pruned or grown in strong sunlight. The cream-coloured flowers appear in spring, with the male flowers boasting red anthers. Separate shrubs produce either male or female flowers, with the female plants producing purple berries.

*Myrsine africana* has been found to have many traditional uses. The Southern Sotho people administer it to rams in order to prevent them covering the ewes before the proper time. The Tswana and Kwena people used a decoction made from the leaves as a 'blood purifier'. In traditional medicine concoctions made from the bark, roots and fruits together with other plants have been used as anthelmintics in humans and livestock. The plant is also traditionally used as an antibacterial agent. In Chinese folklore it is traditionally used to treat diseases such as diarrhoea, rheumatism, toothache, pulmonary tuberculosis and for relieving hemorrhage. It is also used as fragrance in tea, spices, carminative, appetizer and flavouring agent. It has been reported that a decoction of fresh leaves can be taken twice daily for 6-7 days, orally, for the treatment of scanty urination, skin allergies, boils and to purify blood.

Aging of the skin results from two main factors: genetic programming and permanent actinic damage due to environmental stress (UV, detergents, mechanical shocks etc.) with age, especially of people over 40 years. The elasticity of skin is significantly decreased by elastase activity which results in sagging. Histological examination of aged skin reveals a thickened epidermis, an increased number of mast cells and hypertrophic cysts, the infiltration of inflammatory cells, the partial absence and aggregation of elastin fibers and a decrease in collagen fibers in comparison with less aged skin. Biologically, elastase activity significantly increases with age resulting in reduced skin elasticity and in the appearance of wrinkles or stretch marks. Due to the significance of elastase in wrinkle formation of the skin, it is suggested that elastase inhibitors may be beneficial in control of aging of the skin.

The present invention relates to the inhibition of an elastase enyme, porcine pancreatic elastase, by an extract from *Myrsine Africana,* and the use of this extract as an anti-wrinkle active.

## SUMMARY OF THE INVENTION

The present invention provides for use of a topical skin care composition comprising an ethanolic leaf extract of *Myrsine africana* and a dermatologically acceptable vehicle in a cosmetic method of inhibiting elastase activity in skin to reduce the appearance of wrinkles.

The present invention also provides for use of an ethanolic leaf extract of *Myrsine africana* in a cosmetic method of the prevention or treatment of wrinkles in a subject.

The present invention also provides a cosmetic method of inhibiting elastase activity in skin to reduce the appearance of wrinkles, comprising topically applying a topical skin care composition comprising an ethanolic leaf extract of *Myrsine africana* to the skin of a subject in need thereof.

Disclosed herein is a method for preparing a plant extract having elastase inhibitory activity, the method comprising the steps of (i) drying leaves and twigs of a *Myrsine africana* plant, (ii) grinding the dried leaves and twigs, (iii) mixing the ground leaves and twigs with an organic solvent to form a solution, (iv) filtering the solution in order to produce a filtrate; and (v) removing the organic solvent from the filtrate in order to produce the plant extract having elastase inhibitory activity.

The organic solvent may be selected from organic solvents in the group consisting of ethanol, methanol, butanol, water or mixtures thereof. Preferably, the organic solvent is ethanol. The organic solvent is preferably removed from the filtrate by evaporation but could be removed by any other method known to those of skill in the art.

A preservative may be added to the plant extract. Preferably the preservative is a phenoxyethanol and ethylhexylglycerine mixture and preferably about 1% (w/w) of the preservative is added to the plant extract.

The plant extract produced by the method may be prepared in a topical dosage form. The topical dosage form may be selected from the group consisting of an aqueous solution, balm, cream, emulsion, essence, gel, lotion, oil, ointment, spray, sunscreen, suspension or toner.

The plant extract produced by the method may display a 50% inhibitory concentration ($IC_{50}$) of between 26 $\mu$g/mL and 30 $\mu$g/mL when spectrophotometrically assessing the release of p-nitroaniline from N-Succinyl-Ala-Ala-Ala-p-nitroanilide.

Disclosed herein is a topical skin care composition comprising a plant extract of *Myrsine africana* produced according to the method as described herein and a dermatologically acceptable vehicle. The topical skin care composition preferably inhibits elastase activity. The dermatologically acceptable vehicle may be selected from the group consisting of an aqueous solution, balm, cream, emulsion, essence, gel, lotion, oil, ointment, serum, spray, sunscreen, suspension or toner.

Disclosed herein is a topical skin care composition for use in a cosmetic method of inhibiting elastase activity in skin, comprising topically applying the composition to the skin of a subject in need thereof.

Disclosed herein is a plant extract from the plant *Myrsine africana* produced by the methods set forth herein for use in the prevention or treatment of skin aging in a subject. Skin aging may be exhibited as loss of skin elasticity, decrease in collagen, loss or aggregation of elastin fibres, and/or the appearance of wrinkles.

The prevention or treatment of skin aging may be as a result of the inhibition of elastase activity by the plant extract and/or one or more compounds contained therein.

The plant extract may be a leaf or a twig extract. Preferably, the plant extract is a leaf and twig extract. The plant extract may be an organic solvent-derived plant extract. Preferably the organic solvent is selected from the group consisting of ethanol, methanol and butanol or mixtures thereof. Even more preferably the organic solvent is ethanol.

Disclosed herein is a cosmetic method of inhibiting elastase activity in skin, comprising topically applying a topical skin care composition as described herein to the skin of a subject in need thereof.

Disclosed herein is a method of prevention or treatment of skin aging in a subject, comprising topically applying a plant extract produced according to the methods described herein to the skin of the subject.

Also disclosed is a pharmaceutical composition comprising a pharmaceutically effective amount of the crude or purified extract and a pharmaceutically acceptable carrier. In particular the pharmaceutical composition may be administered topically to a subject, preferably a human subject.

## BRIEF DESCRIPTION OF THE FIGURES

Non-limiting embodiments of the invention will now be described by way of example only and with reference to the following figures:

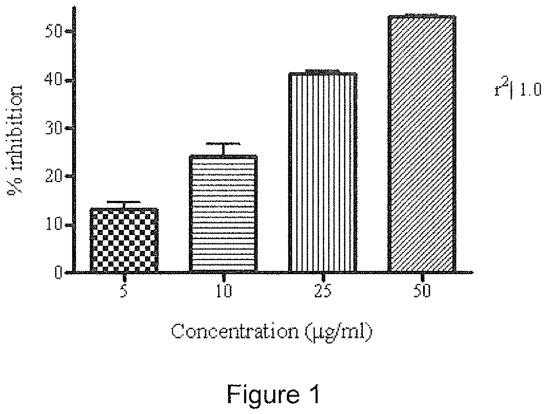

**Figure** 1: Effect of Ursolic Acid on elastase activity for *Myrsine africana* ethanolic leaf extract.

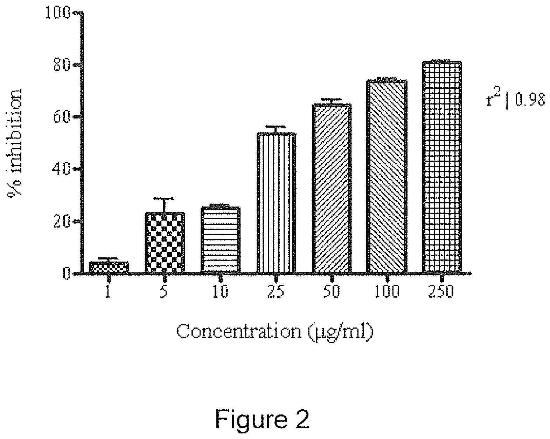

**Figure 2:** Effect of *Myrsine africana* on elastase activity.

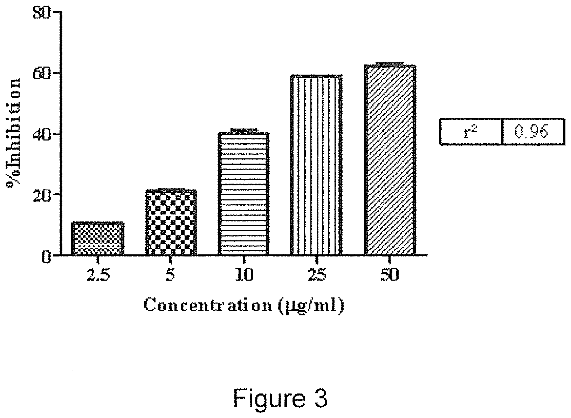

**Figure 3:** Effect of Ursolic Acid on elastase activity for *Myrsine africana* compound MA-NR-01 (Myrsinoside B).

## DETAILED DESCRIPTION OF THE INVENTION

Disclosed herein is a method for preparing plant extracts having elastase inhibitory activity from a *Myrsine africana* plant and topical skin care compositions comprising plant extracts of *Myrsine africana* produced according to the method, specifically plant extracts and compositions for use in cosmetic methods of inhibiting elastase activity in skin. The invention relates to the use and cosmetic methods of use of ethanolic leaf extracts from the plant *Myrsine africana* for prevention or treatment of wrinkles in a subject.

The present invention will now be described more fully hereinafter with reference to the accompanying drawings, in which some, but not all embodiments of the invention are shown.

Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

The disclosure relates to crude or purified extracts derived from the leaves of *Myrsine Africana.* The extracts display elastase inhibitory activity. The extracts are for use in preventing or reducing the appearance of wrinkles and blemishes on the skin.

In the present disclosure porcine pancreatic elastase was used as an enzyme source to set up the inhibition assay. The pancreatic elastase inhibitory activity was determined *in vitro* by the spectrophotometric determination of the release of p-nitroaniline from a synthetic substrate N-Succinyl-Ala-Ala-Ala-p-nitroanilide.

The compound MA-NR-01 (Myrsinoside B) that was isolated from the shoots was also tested *in vitro* for its elastase inhibitory activity. The elastase inhibitory activity is based on the release of p-nitroaniline from a synthetic substrate N-Succinyl-Ala-Ala-Ala-p-nitroanilide. The leaf extract showed good elastase inhibition with a 50% inhibitory

concentration (IC$_{50}$) of 28.04 μg/ml. At a concentration of 125 μg/mL the compound Myrsinoside B was found to have a percentage inhibition of 13.00±1.04.

Mutagenicity was tested using two strains of S. *typhimurium* (TA 98 and TA 100) with no colony growth observed and it was concluded that the *Myrsine africana* ethanolic extract is a non-mutagen. The extract was further found to be a non-irritant during the patch test on human subjects. *Myrsine africana* was found to contain negligible amounts of microorganisms and heavy metals. Gel cream containing the *Myrsine africana* crude extract was found to be stable for two years when kept at temperatures below 30°C. In *in vivo* testing on subjects the test product containing 5% ethanolic extract of M. *africana* was found to be effective in reducing wrinkles after application two times a day for 14 days and 28 days compared to the placebo aqueous cream.

The present invention uses an ethanolic leaf extract of *Myrsine Africana.* It will be understood that the extract may be in the form of a crude extract, a purified extract or a pharmaceutical composition. The extract, pharmaceutical composition may be administered to a patient prior to a symptomatic state associated with skin cancers, or after a symptomatic onset of skin cancer in a subject.

Those skilled in the art will appreciate that there are a number of methods for synthesizing extracts from crude plant material. These methods include, among others, cutting, chopping, macerating and/or grinding raw plant material to at least one solvent in order to obtain a plant extract. It will also be appreciated that the crude plant material may be fresh material or dry plant material.

As used herein the term "crude extract" refers to a concentrated preparation of a plant extract obtained by removing secondary metabolites from the crude plant material with the aid of a suitable solvent. This may be done, for example, by submerging the crude plant material in a suitable solvent, removing the solvent and consequently evaporating all or nearly all of the solvent. As used herein the term "purified extract" refers to an extract obtained by separating the constituent parts of the crude extract from each other. By way of a non-limiting example, the constituent parts of the crude extract may be separated from one another by separating the polar constituents from the non-polar constituents. In so doing the active polar and/or non-polar constituents may thus be concentrated.

As described herein the extract is an extract suitable for topical use on a subject. The subject may include a living animal, preferably a mammal and most preferably a human.

The extract can be prepared in any desired delivery form for example, as a spray, cream, lotion, balm, oil or solid, such as a roll-on, for personal use, or a solid strip. For instance, sprays can be prepared using conventional propellants, such as propane, butane, isobutane, either alone or in various mixtures known to those skilled in the art. Other conventional formulations, including known carriers and additives, will be readily apparent to those skilled in the art.

The pharmaceutical composition containing the extract is in a form suitable for topical use. Suitable forms of the pharmaceutical composition include, for example, gels, lotions, creams, essences, toners, emulsions, soaps, shampoos, rinses, cleansers, solutions, ointments, jellies or suspensions.

The "suitable forms" of the pharmaceutical composition may be combined with "pharmaceutically acceptable carriers" and other elements known in the art to produce creams and lotions for use for general skin care. The pharmaceutical composition may further be combined with other ingredients which promote absorption by the skin.

The extract may be formulated as a pharmaceutical composition by methods known to those skilled in the art. Pharmaceutically acceptable ingredients may be used. The term "pharmaceutically acceptable" refers to properties and/or substances which are acceptable for administration to a subject from a pharmacological or toxicological point of view. Further "pharmaceutically acceptable" refers to factors such as formulation, stability, patient acceptance and bioavailability which will be known to a manufacturing pharmaceutical chemist from a physical/chemical point of view.

By "pharmaceutically acceptable carrier" is meant a solid or liquid filler, diluent or encapsulating substance which may be safely used for the administration of the extract, pharmaceutical composition and/or medicament to a subject.

The use of the extracts or medicaments containing the extract entails administration of an effective amount of the extract or a pharmaceutical composition containing the extract to a subject in order to prevent or treat a condition. The term "effective amount" in the context of preventing or treating a condition refers to the administration of an amount of the active plant extract to an individual in need of treatment, either a single dose or several doses of the extract or pharmaceutical composition containing the extract.

Although some indications have been given as to suitable dosages of the extract and/or pharmaceutical composition containing the extract, the exact dosage and frequency of administration of the effective amount will be dependent on several factors. These factors include the individual components used, the formulation of the extract or pharmaceutical composition containing the extract, the condition being treated, the severity of the condition, the age, weight, health and general physical condition of the subject being treated, and other medication that the subject may be taking, and other factors as are known to those skilled in the art. It is expected that the effective amount will fall within a relatively broad range that can be determined through routine trials.

The following examples are offered by way of illustration and not by way of limitation.

Materials

Elastase [E.C.3.4.21.36] Type IV from Porcine pancreas (E0258, Sigma, USA), N-Succinyl-Ala-Ala-Ala-p-nitroanilide (S4760, Sigma, USA) and the positive control ursolic acid (U001, Natural Remedies Pvt. Ltd., India). Trizma® base: T1503 (Sigma, USA) and hydrochloric acid (H0090, Rankem, India). Ethanol, Tris-HCl buffer, basal medium, Dulbecco's Modified Eagle's Medium (DMEM), murine melanocyte cell line B16-F10, sodium 3-[1-(phenylaminocarbonyl)-3,4-tetrazolium]-bis (4-methoxy-6-nitro benzene sulfonic acid hydrate (XTT), Kojic acid, arbutin, dimethyl sulphoxide (DMSO), N-methyl dibenzopyrazine methyl sulphate (PMS), dextrose, magnesium sulfate ($MgSO_4.H_2O$), citric acid monohydrate, potassium phosphate (diabasic) anhydrous ($K_2HPO_4$), sodium ammonium phosphate ($Na_2NH_2PO_4.4H_2O$), Difco agar, XVB salt solution, 40% glucose, D-biotin, L-histidine, sodium chloride (NaCl), oxoid nutrient broth number 2, sodium monobasic ($NaH_2PO_4.H_2O$), sodium diabasic ($Na_2HPO_4.H_2O$), Oxoid Nutrient broth, 4-nitroquinoline 1-oxide (4-NQO) and Euxyl PE 9010.

## EXAMPLE 1

Plant collection and preparation of ethanol extract

The leaves of *Myrsine africana* were collected during 2011 from the Manie van der Schijff Botanical Garden in Pretoria, South Africa. Plant material (leaves and twigs) was shade dried for two weeks and then ground to a fine powder. The dried powder was macerated in distilled ethanol and extracted for 48 hrs. The filtrate of the plant was collected after filtration with a Buchner funnel. The extract was then collected after the filtrate was put under reduced pressure using a Rotavapor. The extract was kept in a cold room until further use. This extract was used to test for elastase inhibition, cytotoxicity and mutagenicity. The dry ethanolic leaf extract (300 mg) was dissolved in 70% ethanol and used for irritancy, microbial and metal as well physiochemical and *in vivo* efficacy studies.

Isolation of Myrsinoside B

The air-dried and powdered shoots (2.3 kg) were soaked in ethanol to get extract with continuous stirring on a rotatory shaker for 3 days. Dried ethanolic extract of the shoots (88 g) was re-dissolved in ethanol and suspended in 800 ml of distilled water. The water suspension was successively partitioned with petroleum ether, chloroform, ethyl acetate and n-butanol. The organic layers were evaporated on a rotary evaporator to dryness at 40 °C to give different fractions. The EtOAc fraction (18.7 g) was subjected to silica gel column using n-hexane-ethyl acetate-methanol-water, eluents. Similar fractions were pooled based on the TLC profile, which resulted in a total of seven major fractions (MF-1 to MF-7). MF-2 (1.25 g), was subjected to silica gel column with an eluent of dichloromethane: methanol in increasing polarity (0%-100%). Subfraction 2-4 (0.5 g) were chromatographed again on silica gel using DCM:MeOH (7:1), which afforded compound Myrsinoside B (MA-NR-01; 257 mg, 0.0111 %, of dry plant material).

## EXAMPLE 2

Elastase inhibition assay

*Sample preparation*

A sample stock solution of 500 μg/mL and 1120 μg/mL was prepared from M. *africana* leaf extract and compound respectively. Briefly, 2.5 mg of extract and 2.24 mg of the compound was weighed and dissolved in 100 μL of methanol. The volume was then made up to 5 mL for the extract and to 2 mL for the compound with 100mM Tris-HCl buffer, pH 8.0. Subsequent dilutions for the assay were made as required. Compound MA-NR-01 (Myrsinoside B) was isolated using standard procedure as described in Example 1.

*Elastase inhibition assay on ethanolic leaf extract and compound MA-NR-01* (in vitro)

An elastase inhibition assay was carried out largely as per the method of Bieth *et al.* (1974) with modifications. In brief, a pre-incubation mixture contained 100 mM of either Tris-HCl buffer (pH 8.0), vehicle buffer, positive control, or test sample of various concentrations together with 4.942 mU of elastase enzyme. The reagents were mixed and the mixture was pre-incubated at 37°C for 15 min. Following pre-incubation, substrate (N-Succinyl-Ala-Ala-Ala-p-nitroanilide) was added to a final concentration of 0.338mM and the solution was mixed and incubated at 37°C for 30 min. The absorbance was measured at 405nm in a micro-plate reader (Versamax, Molecular devices, USA). A control reaction was carried out without the test sample.

The % inhibition of the enzyme was calculated as follows:

$$\% \text{ inhibition} = \frac{\text{Absorbance (control)} - \text{Absorbance (test)}}{\text{Absorbance (control)}} \times 100$$

$IC_{50}$ was calculated using log-probit analysis.

The leaf extract for *M. africana* was evaluated for its elastase inhibitory activity using the elastase inhibition assay. The assay is based on the release of p-nitroaniline from N-Succinyl-Ala-Ala-Ala-p-nitroanilide that is measured spectrophotometrically. The ethanolic extract of *Myrsine africana* showed promising elastase inhibitory activity with an $IC_{50}$ of 28.04 $\mu$g/mL (Table 1).

The isolated compound MA-NR-01 (Myrsinoside B) was further evaluated for its elastase inhibitory activity. At a concentration of 125 $\mu$g/mL the percentage inhibition of the enzyme was found to be 13.00 $\pm$ 1.04% (Table 1).

**Table 1**: IC$_{50}$ data of test sample (*M. africana*), compound MA-NR-01 and positive control in elastase inhibition assay

| MA ethanolic extract | | | | Compound MA-NR-01 | | | |
|---|---|---|---|---|---|---|---|
| Sample | Concentration (μg/mL) | % Inhibition (Mean ± SEM) | IC$_{50}$ (μg/mL) (95% Confidence Interval | Sample | Concentration (μg/mL) | % Inhibition (Mean ± SEM) | IC$_{50}$ (μg/mL) (95% Confidence Interval |
| Ursolic Acid (Positive control) | 5 | 13.22 ± 2.25 | 40.58 (30.10 - 64.28) | Ursolic Acid (Reference Inhibitor) | 2.5 | 10.64 ± 0.32 | 20.80 (16.51 - 27.53) |
| | 10 | 24.13 ± 4.50 | | | 5 | 21.10 ± 0.53 | |
| | 25 | 41.43 ± 0.92 | | | 10 | 40.07 ± 1.22 | |
| | 50 | 53.16 ± 0.56 | | | 25 | 58.85 ± 0.17 | |
| | | | | | 50 | 62.14 ± 1.18 | |
| *M. africana* | 1 | 4.29 ± 2.43 | 28.04 (22.52 - 35.06) | MA-NR-01 | 7.8125 | 11.79 ± 4.35 | -** |
| | 5 | 22.87 ± 9.82 | | | 15.625 | 4.79 ± 1.66 | |
| | 10 | 25.21 ± 1.55 | | | 31.25 | 6.76 ± 4.57 | |
| | 25 | 53.36 ± 4.78 | | | 62.5 | 5.73 ± 1.04 | |
| | 50 | 64.58 ± 3.57 | | | 125 | 13.00 ± 1.04 | |
| | 100 | 73.36 ± 1.96 | | | 250 | 25.75 ± 1.46 | |
| | 250 | 81.19 ± 0.91 | | | 500 | 31.02 ± 1.22 | |

**EXAMPLE 3**

Mutagenicity

*Preparation of glucose solution (40%)*

In a 1 L flask, 1 L of distilled water was added to 400 g of dextrose. The solution was placed on a magnetic stirrer until clear. The flask was then autoclaved for 20 min at 121°C. The solution was then stored at 4°C (refrigerated).

*Preparation of salt solution XVB (Vogel-Bonner medium E (50x))*

Warm distilled water (650 mL) was added to 10 g of magnesium sulfate ($MgSO_4.H_2O$), 100 g of citric acid monohydrate, 500 g potassium phosphate (diabasic) anhydrous ($K_2HPO_4$) and 175 g of sodium ammonium phosphate ($Na_2NH_2PO_4.4H_2O$) in a 2 L flask on a magnetic stirrer, making sure that before each new salt was added the former was completely dissolved. Distilled water was then added to a final volume of 1 L. The solution was then autoclaved for 30 min at 121°C, and thereafter allowed to cool and stored in the dark at room temperature.

*Preparation of the glucose minimal agar plates*

Difco agar (15 g) was added to 900 mL of distilled water, after which solution was then autoclaved for 30 min at 121°C and allowed to cool to approximately 65°C. Thereafter, 20 mL of the sterile XVB salt solution was added to the flask ensuring thorough mixing. Finally, 50 mL of the 40% glucose solution was added to the same flask, ensuring thorough mixing. The mixture was then dispensed into 100 x 15 mm petri plates ($\pm$ 25-30 mL of agar in each plate). The plates were allowed to solidify before being placed in sealed plastic bags and stored at 4°C overnight. The following day the plates were allowed to reach room temperature before being used in the test.

*Preparation of the histidine/biotin solution (0.5 mM)*

Distilled water (1 L) was brought to the boil and 124 mg of D-biotin and 96 mg of L-histidine were dissolved in the water.

*Preparation of the supplemented top agar*

Six grams of agar and 6 g of NaCl were added to 900 mL of distilled water. The mixture was heated in an autoclave for 10 min to melt the agar. Following this, 100 mL of the previously prepared Histidine/biotin solution was added to the jar. The top agar was labelled and autoclaved for a further 30 min and stored at room temperature in the dark.

*Preparation of nutrient broth for growth of tester strains*

Oxoid nutrient broth number 2 (25 g) was added to 1 L of distilled water and stirred until the broth was dissolved. A quantity of the broth (50 mL) was dispensed into an Erlenmeyer flask and autoclaved for 20 min. The solution was cooled and stored in a dark area at room temperature.

*Preparation of sodium phosphate buffer 0.1mM, pH 7.4*

$NaH_2PO_4.H_2O$ (13.8 g) was added to 1 L of water (sodium monobasic, 0.1 M). Thereafter, 14.2 g of $Na_2HPO_4.H_2O$ was added to 1 L of water (sodium diabasic, 0.1 M). Then 120 mL of sodium diabasic solution was added to 880 mL of monobasic solution and mixed. The pH was adjusted to 7.4 by addition of the 0.1 M sodium diabasic solution. The buffer was autoclaved for 30 min at 121°C. The bottle was allowed to cool before being stored at room temperature in a dark place.

*Method for test plates*

The sample was cultured and tested at 3 different concentrations with each test being conducted in triplicate. 100 $\mu$L of bacterial stock was incubated in 20 mL of Oxoid Nutrient broth for 16 h at 37°C on a rotative shaker. 0.1 mL of this overnight culture was added to 2.0 mL of top agar (containing traces of biotin and histidine) together with 0.1 mL test solution (test sample, solvent control or positive control) and 0.5 mL phosphate buffer. The top agar mixture was poured over the surface of a minimal agar plate and incubated for 48 h at 37°C. After incubation the number of revertant colonies (mutants) was counted. All cultures were performed in triplicate (except the solvent control where five replicas were performed). Toxicity can be checked by investigation of the background layer of bacteria. Absence of toxicity was examined by observing the background bacterial growth, which should normally be present. The positive control used in this study was 4-nitroquinoline 1-oxide (4-NQO) at a concentration of 2 $\mu$g/mL.

The ethanolic extract of *M. africana* was applied to two different strains of bacteria according to the number of colonies. According to the results obtained M. *africana* did not display any mutagenic effects in either TA 98 or TA 100 strains of S. *typhimurium* (Table 2). *M. africana* did not display a ratio of 2 or higher in terms of revertants and no dose response curve was observed. The number of colonies observed may have been affected by the possibility that *M. africana* has antibacterial properties.

**Table 2:** Average amount of colonies of *S. typhimurium* observed at the indicated concentrations of MA

| Concentration of test sample (mg/mL) | Number of colonies | |
|---|---|---|
| | TA 98 | TA 100 |
| 5 | 18.67$\pm$1.15 | 103.33$\pm$2.52 |
| 0.5 | 20.00$\pm$3.46 | 105.33$\pm$1.53 |
| 0.05 | 15.33$\pm$2.08 | 104.00$\pm$3.00 |
| Positive control (4NQO) | 168.33$\pm$9.81 | 438.67$\pm$9.07 |
| Blank | 26.00$\pm$3.46 | 112.40$\pm$3.65 |

## EXAMPLE 4

Microbial count, metal analysis and irritancy testing (patch test)

The M. *africana* ethanol extract was prepared as previously described in order to carry out the microbial count, metal analysis and irritancy testing.

For the determination of TMA (total microbial activity) 50 mL of M. *africana* extract was sent to Swift Micro Laboratories for determination of bacterial, yeast and mould count. Results were reported in colony forming units per millilitre (CFU/mL).

For determination of metal contaminants 50 mL of *M. africana* extracts were sent to Microchem Laboratories. Metal contaminant detection was done for arsenic, mercury and lead (mg/100 g).

A sample of the ethanol extract was applied on the inner forearm of twenty (20) volunteers aged between 19-65 years using patch discs for 24 hours to determine irritancy. Visual assessments of the areas were made after 24, 48, 72 and 96 hours. Any reaction was graded as a score of 0, 0.5, 1, 2, 3, and 4, where 0 represented a no response and 4 a fiery redness, papules, oedema or bullae. The forearm of each volunteer included a positive control of sodium lauryl sulphate solution (1 g/100 mL) and a negative control of demineralised water.

Medicinal plants may be contaminated with a broad range of microbial contaminants, such as bacteria, fungi and viruses. This is why it has become important to assess microbial load of medicinal plants for quality control purposes (Kneifel *et al.,* 2002). The TMA (total microbial activity) was found to have no activity since no growth of microbes was observed (Table 3). Also no growth of yeast or mould was observed for *M. africana* ethanolic extract.

Toxic metals are defined as those which have no known function in the body and once present are harmful. These toxic metals have become a great concern as they are the causative agent of illness, aging and even genetic defects (Wilson, 2011). Low levels the heavy metals, arsenic, cadmium, mercury and lead were observed for *M. africana* ethanolic extract (Table 4).

The leaf extract of *M. africana* was found to be non-irritant since the irritancy potential was found to be -9.00% compared to the positive control sodium lauryl sulphate with a irritancy potential of 100% (Table 5).

**Table 3:** Microbial count of *M. africana* ethanolic extract

| Test type | Method No. | Result | Limits |
|---|---|---|---|
| **TMA** | SWJM 35 | No growth cfu/mL | N/A |
| **Yeast** | SWJM 50 | No growth cfu/mL | N/A |
| **Mould** | SWJM 50 | No growth cfu/mL | N/A |

**Table 4:** Heavy metal analysis of *M. africana* ethanolic extract

| Test type | MOU% | Result |
|---|---|---|
| Arsenic (mg/100g) | 9.77 | <0.003 |
| Cadmium (mg/100g) | 5.40 | <0.001 |
| Mercury (mg/100g) | 7.94 | <0.010 |
| Lead (mg/100g) | 6.22 | <0.005 |

**Table 5:** Irritancy patch test results after ninety-six (96) hours

| Test product name | Average value | Average score | Number of subjects with reactions after 48 hours | Irritancy potential % (TP-NC)/(PC-NC) | Irritancy |
|---|---|---|---|---|---|
| *M. africana* ethanolic extract | 0.21 | 0.54 | 6 | -9.00 | Non irritant |
| Positive Control Right | 0.54 | 1.03 | 12 | 100 | Irritant |

## EXAMPLE 5

Preservative challenge test

The sample was tested in accordance with USP 31 method (USPC 2008), using: *Pseudomonas aeruginosa, Staphylococcus aureus, Escherichia coli, Candida albicans* and *Aspergillus niger* in a preservative challenge test. The preservative Euxyl PE 9010 was used as the positive control.

The growth of organisms *Pseudomonas aeruginosa, Staphylococcus aureus, Escherichia coli, Candida albicans* and *Aspergillus niger* was negligible when *M. africana* ethanolic extract together with a standard preservative was exposed to these organisms. This is an indication that *M. africana* ethanolic extract passed the preservative challenge test (Table 6).

**Table 6:** Preservative challenge test of *M. africana* ethanolic extract against *Pseudomonas aeruginosa, Staphylococcus aureus, Escherichia coli, Candida albicans* and *Aspergillus niger.*

| Exposure time | Number of organisms recovered per mL/gram | | | | |
|---|---|---|---|---|---|
| Number of viable organisms added at zero time | *E. coli* $8.8 \times 10^6$ | *P. aerug* $8.4 \times 10$ | *S. aureus* $1.0 \times 10^6$ | *C. albicans* $3.1 \times 10^6$ | *A.niger* $1.9 \times 10^6$ |
| 7 Days | NEG | NEG | NEG | NEG | NEG |
| 14 Days | NEG | NEG | NEG | NEG | NEG |
| 21 Days | NEG | NEG | NEG | NEG | NEG |
| 28 Days | NEG | NEG | NEG | NEG | NEG |

## EXAMPLE 6

Physiochemical analysis

*Determination of odour and colour of the extracts*

The odour and colour (appearance) of the *M. africana* extract was recorded visually.

*Determination of pH*

The pH of the *M. africana* extract was determined using a pH meter. The pH meter was calibrated using the standard pH4 and pH7 standard solutions. The electrode was then submerged in the extracts and recorded.

*Determination of specific gravity of the extracts*

The specific gravity of the extracts was determined using a pycnometer. The pycnometer was weighed while it was empty. It was then filled with distilled water and weighed again. The weight before filling was subtracted from the weight after filling and divided by the capacity (mL) of the pycnometer to give a specific gravity in g/mL.

*Determination of viscosity of gel cream formulations*

Determination of the viscosity of the *M. africana* was done using a viscometer (Brookfield). The size 5 spindle at a speed of 5 rpm was used to obtain a viscometer reading. The viscometer reading was multiplied by the factor and the gel cream viscosity was recorded in cPs (Centiposes).

*Determination of refractive index of extracts*

The refractive index was determined using a refractometer. A glass Pasteur Pipette was used to suck up 2-3 mL of the plant extract. The plant extract was placed on the refractometer lens and the refractive index was determined at 25°C.

The results of the physicochemical analysis are presented in Table 7.

**Table 7:** Physiochemical analysis of *M. africana* ethanolic extract

| Physiochemical parameter | Value |
| --- | --- |
| pH | 5.28 |
| Viscosity | 1 centipoise (cP) |
| Refractive index (RI) | 1.35134 |
| Appearance | Dark brown, clear liquid |
| Loss of weight on drying | 50% |
| Odour | Characteristic, Berry-juice like |
| Solubility | Soluble in water |
| Specific gravity (SG) | 0.9848 |
| Heavy metal content*: arsenic, mercury and lead | Not detected |
| Total microbial Activity* | 9 CFU/mL |
| Yeast and mould * | 6 CFU/ mL |

**EXAMPLE 7**

Stability test

*M. africana ethanolic crude extract*

Four 250 mL glass jars containing 200 mL of extract were labelled and stored at their specific temperatures. The preservative Euxyl PE 9010 was added to each jar at 1%. The stability test was performed at 5°C, 25°C, 40°C and 50°C. Various parameters of the extract stored at each temperature were measured after 1, 2, 4, 8 and 12 weeks. The parameters included appearance and odour which were both recorded sensory. pH was measured using a pH meter. Specific gravity was measured using a pycnometer was only measured initially and at the final 12 week interval. For all the samples the initial result for each parameter was recorded and then for all subsequent intervals recordings were taken as well.

*Gel cream and M. africana ethanolic extract*

Four 250 mL glass jars containing 200 mL of cream gel were labeled and stored at their specific temperatures. The stability test was performed at 5°C, 25°C, 40°C and 50°C. Various parameters of the cream gel were stored at each temperature were measured after 1, 2, 4, 8 and 12 weeks. The parameters included appearance and odour which were recorded using sensory methods. pH was measured using a pH meter. Specific gravity was measured using a pycnometer and viscosity was measured using a viscometer. For all the samples the initial result for each parameter was recorded and then for all subsequent intervals recordings were taken as well.

Results taken at week 0 were compared to those based on observations at the end of 12 weeks. We expect to see a smaller change in the extracts at the lower temperatures and more adverse effects in the samples stored at the extreme temperatures. The initial pH value for the ethanolic *M. africana* extract was 5.28 and dropped slightly to 4.99, 4.97, 5.03 and 5.12 for the 5°C, 25°C, 40°C and 50°C samples respectively (Table 8). From the results, it looks like the drop in pH is time related and temperature related, as the samples stored at accelerated conditions of temperature show a slower drop than the samples stored at ambient and 5°C. Specific gravity of the extracts which was only performed at the end of the 12 week interval had an initial value of 0.9848 g/mL and dropped to 0.853, 0.782, 0.816 and 0.850 g/mL for the 5°C, 25°C, 40°C and 50°C samples respectively. This could most likely be due to sedimentation of the sample (Table 8).

Darkening of the product was recorded from the first week of storage at elevated temperatures and after 12 weeks at ambient conditions. The darkening of the product is due to the extract and the overall efficacy of the product may be affected (Table 8). The darkening may be just a physical change or a chemical change due to the temperature. However, darkening of products using extract is a normal occurrence. pH and viscosity show a drop in the values at accelerated conditions of temperature but these will not affect the overall product performance.

Based on the above results a 2 year shelf life can be predicted for this product. The product should be stored away from direct sunlight and at temperatures below 30°C.

**Table 8:** Stability test of *M. africana* ethanolic crude extract and gel cream containing M. *africana* ethanolic crude extract

| Parameters observed | Storage conditions | MA ethanolic extract | | Gel cream | |
| --- | --- | --- | --- | --- | --- |
| | | Storage intervals | | Storage intervals | |
| | | Week 0 | Week 12 | Week 0 | Week 12 |
| Appearance | 5 °C | Dark brown liquid (with red tint) | Complied | Light brown gel cream | Complied |
| | 25 °C | | Complied | | Darker than std |
| | 40 °C | | Complied | | Darker than std |
| | 50 °C | | Complied | | Darker than std |
| Odour | 5 °C | Characteristic berry like odour | Complied | Characteristic berry like odour | Complied |
| | 25 °C | | Strong alcohol odour | | Complied |
| | 40 °C | | Strong alcohol odour | | Complied |
| | 50 °C | | Strong alcohol odour | | Complied |
| pH (25°C) | 5 °C | 5.28 | 4.99 | 6.07 | 5.84 |
| | 25 °C | | 4.97 | | 5.95 |
| | 40 °C | | 5.03 | | 5.91 |
| | 50 °C | | 5.12 | | 5.80 |

(continued)

| Parameters observed | Storage conditions | MA ethanolic extract | | Gel cream | |
|---|---|---|---|---|---|
| | | Storage intervals | | Storage intervals | |
| | | Week 0 | Week 12 | Week 0 | Week 12 |
| Viscosity (25°C) Spindle: 5 at 5 rpm Factor: 800 | 5 °C | N/A | N/A | 45 600 cPs | 48 000 |
| | 25 °C | | | | 45 440 |
| | 40 °C | | | | 35 200 |
| | 50 °C | | | | 28 800 |
| Specific Gravity (25°C) | 5 °C | 0.9848 g/mL | 0.853 | 0.9866 g/mL | 0.969 |
| | 25 °C | | 0.782 | | 0.965 |
| | 40 °C | | 0.816 | | 0.972 |
| | 50 °C | | 0.850 | | 0.971 |

**EXAMPLE 8**

*M. Africana ethanolic leaf extract in vivo subject efficacy testing*

The dry ethanolic leaf extract (300 mg) was dissolved in 70% ethanol (40 mL), whereafter the concentration of the product was made with a 5% dilution.

A group of twenty-two (22) subjects were recruited and the procedure of testing was explained to them verbally and a form of consent and medical history was signed by each subject. Personal details and each subject's assessment of their own skin type were recorded. Colour photographs were taken at each time interval to serve as a recording of the study conducted. All subjects were instructed to rest for 20 min before any testing was conducted. The designated left and right side of the test site was cleansed with a standard eye make-up remover and allowed to air dry for 3 min. The temperature and relative humidity were recorded during the time of the study. No test sites were demarcated on the face as CSI software needed the complete wrinkle length for analysis. Product application was randomised according to the test substance sequence. A photo was taken of the region of interest for the Visio scan analysis as well. An operator trained the test subjects in applying the test products evenly to the test sites, according to the test substance sequence. Subjects were restricted from using any topical products or any medication not approved by the study sponsor for the duration of the study. Baseline (BL) photos were taken before application of test products and again on day fourteen (D14) and day twenty-eight (D28) to serve as a recording of the study. Subjects were given test product to apply twice a day. Subjects returned to the testing facility on day fourteen (D14) as well as day twenty-eight (D28) after baseline procedure. A placebo of aqueous cream was also used.

The Visioscan and Visioface were gently sterilised with 70% Ethanol and a tissue between each reading.

Visioface works on the whole wrinkle length, whereas Visioscan only works on sections of the wrinkle.

*Visioscan - wrinkle*

A t-Test was performed to compare the test product sites and the untreated control sites at baseline (thus before application of test products) and no statistical significant difference was found between the two test sites on a 5% level of confidence. A t-Test was performed to determine the treatment effect by comparing the average difference values between baseline to day fourteen (D14-BL) of the test product and the placebo control sites and there was no statistical significance difference on a 5% level of confidence. A t-Test was performed to determine the treatment effect by comparing the average difference values between baseline to day twenty-eight (D28-BL) of the test product and the placebo control sites and there was a statistical significance difference on a 5% level of confidence. The values of the test product FCAH106/4325 was significantly lower than the values for FCAH106/AqC (Table 9).

*Visioface (CSI) - Wrinkle*

A t-Test was performed to compare the test product sites and the untreated control sites at baseline (thus before application of test products) and no statistical significant difference was found between the two test sites on a 5% level of confidence. A t-Test was performed to determine the treatment effect by comparing the average difference values between baseline to day fourteen (D14-BL) of the test product and the placebo control sites and there was a statistical significance difference on a 5% level of confidence. The values of the test product FCAH106/4325 was significantly lower than the values for FCAH106/AqC A t-Test was performed to determine the treatment effect by comparing the average difference values between baseline to day twenty-eight (D28-BL) of the test product and the placebo control sites and there was a statistical significance difference on a 5% level of confidence. The values of the test product FCAH106/4325 was significantly lower than the values for FCAH106/AqC (Table 9).

Table 9: Comparison of Visioscan (wrinkle) and Visioface - CSI (wrinkle) values for the test product FCAH106/4325 compared to the placebo control FCAH106/AqC test sites. Mean ($\pm$ Standard Deviation)

| Days | | FCAH106/4325 | FCAH1 06/AqC | (Diff) p-Value |
|---|---|---|---|---|
| **Baseline** | Visioscan (wrinkle) | 68.24 ($\pm$ 10.39) | 65.56 ($\pm$ 7.43) | 0.160 |
| | Visioface - CSI (wrinkle) | 14.45 ($\pm$ 2.98) | 14.10 ($\pm$ 2.51) | 0.344 |
| **Day 14 - baseline** | Visioscan (wrinkle) | 0.13 ($\pm$ 5.92) | -0.46 ($\pm$ 5.07) | 0.360 |
| | Visioface - CSI (wrinkle) | -1.15 ($\pm$ 1.39) | 0.50 ($\pm$2.14) | 0.002 |
| **Day 28 - baseline** | Visioscan (wrinkle) | -2.74 ($\pm$ 7.49) | 1.16 ($\pm$ 5.94) | 0.026 |
| | Visioface - CSI (wrinkle) | -2.20 ($\pm$ 1.91) | 0.90 ($\pm$ 2.05) | <0.001 |

Conclusions

The examples above show that the ethanolic leaf extract of *Myrsine africana* exhibits promising elastase inhibitory activity. The isolated compound MA-NR-01 (Myrsinoside B) was found to have a percentage inhibition of 13.00 $\pm$ 1.04% at a concentration of 125 μg/mL.

It is shown that the ethanolic leaf extract of *M. africana* inhibits the growth of *S. typhimurium* and is therefore considered not to be mutagenic.

No microbial growth was observed for *M. africana* and it is concluded that the sample contains an amount below the limit. It was found that *M. africana* ethanolic extract contained an amount below the limit of heavy metals allowed. According to clinical studies the ethanolic extract of *M. africana* was found to be a non-irritant on human skin.

No significant growth was observed of the test organisms: *Pseudomonas aeruginosa*, *Staphylococcus aureus*, *Escherichia coli*, *Candida albicans* and *Aspergillus* niger during the preservative challenge test.

The physiochemical parameters that were tested substantiated the potential use of *M. africana* in a cosmeceutical for the treatment of wrinkles.

It was concluded through accelerated stability testing that cream gel containing *M. africana* ethanolic crude extract had a shelf life of 2 years when stored away from direct sunlight at temperatures below 30°C.

In the human subject testing it was found that for the Visioscan (wrinkle) that the test product FCAH106/4325 was effective in reducing wrinkles after twenty-eight days (D28) of consecutive application (twice a day) when compared to the placebo control (FCAH106/AqC). For the Visioface - CSI (wrinkle) test it was found that the test product FCAH 106/4325 was effective in reducing wrinkles after fourteen days (D14) and twenty-eight days (D28) of consecutive application (twice a day) when compared to the placebo control (FCAH106/AqC). Since Visioface looked at each subject's Crow's Feet Area in a whole, therefore considering depth and length of wrinkle, it can be concluded that *M. africana* passed wrinkle tests on day 14.

**REFERENCES**

Beith J, Spiess B, Wermuth CG (1974). The synthesis and analytical use of a highly sensitive and convenient substrate of elastase. Biochem. Med., 11(4): 350-357.

Kneifel W, Czech E, Kopp B (2002) Microbial contamination of medicinal plants - a review. Planta Medica, 68(1): 5-15.

Wilson L (2011) Toxic Metals and Human Health. The Centre for Development. [Online].

## Claims

1. Use of a topical skin care composition comprising an ethanolic leaf extract of *Myrsine africana* and a dermatologically acceptable vehicle in a cosmetic method of inhibiting elastase activity in skin to reduce the appearance of wrinkles.

2. Use of an ethanolic leaf extract of *Myrsine africana* in a cosmetic method of the prevention or treatment of wrinkles in a subject.

3. The use of claim 2, wherein the prevention or treatment of wrinkles is as a result of the inhibition of elastase activity by the ethanolic leaf extract and/or one or more compounds contained therein.

4. A cosmetic method of inhibiting elastase activity in skin to reduce the appearance of wrinkles, comprising topically applying a topical skin care composition comprising an ethanolic leaf extract of *Myrsine africana* to the skin of a subject in need thereof.

5. The cosmetic method of claim 4, wherein the topical skin care composition comprises a dermatologically acceptable vehicle.

## Patentansprüche

1. Verwendung einer topischen Hautpflegezusammensetzung, umfassend einen ethanolischen Blattextrakt von *Myrsine africana* und ein dermatologisch unbedenkliches Vehikel bei einem kosmetischen Verfahren zur Inhibierung von Elastaseaktivität in der Haut zur Verringerung des Auftretens von Falten.

2. Verwendung eines ethanolischen Blattextrakts von *Myrsine africana* bei einem kosmetischen Verfahren zur Prävention oder Behandlung von Falten bei einem Individuum.

3. Verwendung nach Anspruch 2, wobei die Prävention oder Behandlung von Falten sich als Ergebnis der Inhibierung von Elastaseaktivität durch den ethanolischen Blattextrakt und/oder eine oder mehrere darin enthaltene Verbindungen ergibt.

4. Kosmetisches Verfahren zur Inhibierung von Elastaseaktivität in der Haut zur Verringerung des Auftretens von Falten, umfassend das topische Aufbringen einer topischen Hautpflegezusammensetzung, die einen ethanolischen Blattextrakt von *Myrsine africana* umfasst, auf die Haut eines Individuums, bei dem diesbezüglicher Bedarf besteht.

5. Kosmetisches Verfahren nach Anspruch 4, wobei die topische Hautpflegezusammensetzung ein dermatologisch unbedenkliches Vehikel umfasst.

## Revendications

1. Utilisation d'une composition topique de soin de la peau comprenant un extrait éthanolique de feuilles de *Myrsine africana* et un véhicule acceptable sur le plan dermatologique dans un procédé cosmétique d'inhibition de l'activité d'élastase dans la peau pour réduire l'apparition de rides.

2. Utilisation d'un extrait éthanolique de feuilles de *Myrsine africana* dans un procédé cosmétique de la prévention ou du traitement de rides chez un sujet.

3. Utilisation selon la revendication 2, la prévention ou le traitement de rides étant le résultat de l'inhibition de l'activité d'élastase par l'extrait éthanolique de feuilles et/ou un ou plusieurs composés contenus dans celui-ci.

4. Procédé cosmétique d'inhibition de l'activité d'élastase dans la peau pour réduire l'apparition de rides, comprenant une application de manière topique d'une composition topique de soin de la peau comprenant un extrait éthanolique de feuilles de *Myrsine africana* à la peau d'un sujet qui en a besoin.

EP 3 386 596 B1

**5.** Procédé cosmétique selon la revendication 4, la composition topique de soin de la peau comprenant un véhicule acceptable sur le plan dermatologique.

Figure 1

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MANIE VAN DER SCHIJFF.** *Botanical Garden in Pretoria,* 2011
- **BEITH J ; SPIESS B ; WERMUTH CG.** The synthesis and analytical use of a highly sensitive and convenient substrate of elastase. *Biochem. Med.,* 1974, vol. 11 (4), 350-357
- **KNEIFEL W ; CZECH E ; KOPP B.** Microbial contamination of medicinal plants - a review. *Planta Medica,* 2002, vol. 68 (1), 5-15
- **WILSON L.** Toxic Metals and Human Health. The Centre for Development, 2011